(19) Europäisches Patentamt
European Patent Office
Office européen des brevets

(11) EP 2 972 523 B1

# (12) EUROPEAN PATENT SPECIFICATION

(45) Date of publication and mention of the grant of the patent:
**16.08.2017 Bulletin 2017/33**

(21) Application number: **14713971.1**

(22) Date of filing: **28.02.2014**

(51) Int Cl.:
***G02B 1/04*** *(2006.01)*

(86) International application number:
**PCT/US2014/019467**

(87) International publication number:
**WO 2014/149546 (25.09.2014 Gazette 2014/39)**

(54) **SILICONE-CONTAINING CONTACT LENS HAVING CLAY TREATMENT APPLIED THERETO**

SILIKONHALTIGE KONTAKTLINSE MIT DARAUF ANGEWENDETER TONBEHANDLUNG

LENTILLE DE CONTACT À TENEUR EN SILICONE AYANT UN TRAITEMENT PAR ARGILE APPLIQUÉ À CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361786903 P**
**24.02.2014 US 201414187578**

(43) Date of publication of application:
**20.01.2016 Bulletin 2016/03**

(73) Proprietor: **Johnson & Johnson Vision Care Inc.**
**Jacksonville, FL 32256 (US)**

(72) Inventors:
- **VANDERLAAN, Douglas G.**
**Jacksonville, Florida 32206 (US)**
- **PATTON, Jaqunda**
**Jacksonville, Florida 32218 (US)**
- **VENKATASUBBAN, Kunisi**
**Jacksonville, Florida 32224 (US)**

(74) Representative: **Carpmaels & Ransford LLP**
**One Southampton Row**
**London WC1B 5HA (GB)**

(56) References cited:
**EP-A1- 2 253 590 WO-A2-02/096477**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 972 523 B1

## Description

### Related Applications

This application claims the benefit of U.S. Patent Application Serial No. 14/187,578, filed February 24, 2014, entitled SILICONE-CONTAINING CONTACT LENS HAVING CLAY TREATMENT APPLIED THERETO; and U.S. Provisional Patent Application Serial No. 61/786,903, filed March 15, 2013, entitled SILICONE-CONTAINING CONTACT LENS HAVING CLAY APPLIED TO THE SURFACE.

### Field of the Invention

The present invention relates to a silicone-containing contact lens having clay applied to the surface.

### Background of the Invention

Contact lenses have been used commercially to improve vision since the 1950s. The first contact lenses were made of hard materials. Although these lenses are still currently used, they are not suitable for all patients due to their poor initial comfort and their relatively low permeability to oxygen. Later developments in the field gave rise to soft contact lenses, based upon hydrogels, which are extremely popular today. Many users find soft lenses are more comfortable, and increased comfort levels can allow soft contact lens users to wear their lenses longer than users of hard contact lenses.

Silicone hydrogel materials have proven to be very successful contact lens materials. They are typically formed by copolymerizing a mixture of silicone-containing monomers or macromers with hydrophilic monomers. The amount of water absorbed by the final hydrated material can be controlled by selecting the type and amount of hydrophilic monomer or monomers. Some silicone hydrogels have wettable surfaces, and others have surfaces with poor wettability, even when the water content of the hydrated material is relatively high.

If the surface of a silicone hydrogel material has poor wettability, then surface treatment is typically required in order to make it suitable for use in a contact lens. Silicone hydrogels that are wettable with or without surface treatment, such as those disclosed in US Patent No. 7,052,131.

WO 02/096477 A2 concerns diffusion-controllable coatings for medical devices, where the coating is capable of controlling the out-diffusion of guest materials from the medical device.

EP 2253590 A1 concerns an organosol containing magnesium fluoride hydroxide.

Clays have been added to various polymeric articles, particularly in the food packaging area, to decrease the permeability of various components through the resulting polymeric article. For example, montmorillonite clays have been added to food packaging plastics to decrease the gas permeability of the polymers. Clays have also been used to treat plastic articles, including contact lenses to slow the rate of a diffusible material, such as a drug from a contact lens. In each of these cases it is believed that the clay decreases permeability by creating a tortuous path for the gas or diffusible material. It has now been surprisingly found that the wettability of the surface of a silicone containing contact lens can be improved by applying a clay to the surface of the contact lens.

### Summary of the Invention

In one aspect, the present invention relates to a contact lens including at least one clay and at last one silicone component, wherein the at least one clay is applied to the surface of the contact lens and the contact lens does not include any diffusible material whose release from the contact lens is inhibited by the at least one clay.

In another aspect, the present invention relates to a method of improving the wettability of a contact lens, said method comprising applying, without pretreatment, at least one clay to at least a portion of at least one surface of said contact lens, wherein said contact lens comprises at least one silicone component.

In another aspect, the present invention relates to a method of comprising applying, without pretreatment, at least one clay to at least a portion of at least one surface of a contact lens comprising at least one silicone component.

In another aspect, the present invention features a manufacturing a contact lens by applying at least one clay to the surface of the contact lens, wherein the contact lens includes at last one silicone component and the contact lens does not include any diffusible material whose release from the contact lens is inhibited by the at least one clay

Other features and advantages of the present invention will be apparent from the detailed description of the invention and from the claims.

### Detailed Description of the Invention

It is believed that one skilled in the art can, based upon the description herein, utilize the present invention to

its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

Definitions

As used herein, the term "diffusible material" as used herein is a material included within the contact lens which is intended to diffuse out of the contact lens and impart a desired functionality to the contact lens, such as drugs, low molecular weight, non-reactive wetting agents, and monomers and macromer which are not polymerized during the curing process. Other diffusible materials are disclosed in US Patent No. 7,666,461. Diffusible materials do not include water or osmolality or pH adjusting agents within the lens or its storage solution. In one embodiment, the contact lens does not comprise any diffusible material whose release from said lens is inhibited by said clay. In one embodiment, the contact lens does not comprise any diffusible materials.

As used herein, "osmolality adjusting agents" are inorganic salts which are used to adjust the osmolality of a packaging or storage solution. Examples include sodium chloride, sodium sulfate, sodium acetate, sodium citrate, sodium borate, sodium phosphate, sodium hydrogenphosphate, sodium dihydrogenphosphate, sodium lactate or the corresponding potassium, calcium or magnesium salts of the same and the like. Non-ionic compounds may also be used to provide the desired osmolality. Suitable non-ionic compounds include polyethylene glycols, polyhydroxy compounds, polyether compounds, sugars, polyvinylamides (such as PVP, PVMA), dextrans, cyclodextrans and mixtures thereof and the like.

As used herein, "pH adjusting agents" include NaOH, HCl, buffers including borate and phosphate buffers.

As used herein "release" is the eluting of a component from the contact lens by diffusion.

Clays have been added to polymeric materials to provide barrier coatings. Such coatings are generally multilayer in order to create offset layers of plate-like clay materials which create a tortuous path and slow or inhibit diffusion. As used herein "inhibit" means a reduction in diffusion of at least about 50%, 20% or 10%.

As used herein "silicone hydrogel" means a polymer formed from at least one silicone containing component and at least one hydrophilic component. Generally silicone hydrogels have water contents of at least about 10%, and about 20 to about 70%.

As used herein "reactive mixture" refers to the mixture of components (both reactive and non-reactive) which are mixed together and subjected to polymerization conditions to form the hydrogels and contact lenses of the present invention. The reactive mixture comprises reactive components such as monomers, macromers, prepolymers, cross-linkers, and initiators, and additives such as wetting agents, release agents, dyes, light absorbing compounds such as UV absorbers, and photochromic compounds, any of which may be reactive or non-reactive but are capable of being retained within the resulting contact lens, as well as pharmaceutical and nutriceutical compounds, and any diluents.

As used herein, the term "contact lens" refers to ophthalmic devices that reside on the eye. These devices can provide optical correction, cosmetic enhancement, UV blocking and visible light or glare reduction, therapeutic effect, including wound healing, delivery of drugs or neutraceuticals, diagnostic evaluation or monitoring, or any combination thereof. The term contact lens includes, but is not limited to, soft contact lenses, hard contact lenses, overlay lenses, ocular inserts.

Concentrations of components of the reactive mixture are given herein in weight % of all components in the reaction mixture, excluding any diluents. When diluents are used, their concentrations are given herein as weight % based upon the amount of all components in the reaction mixture and the diluents.

Silicone Component

A silicone-containing component (or silicone component) is one that contains at least one [-Si-O-Si] group, in a monomer, macromer or prepolymer. In one embodiment, the Si and attached O are present in the silicone-containing component in an amount greater than 20 weight percent, such as greater than 30 weight percent of the total molecular weight of the silicone-containing component. Useful silicone-containing components include polymerizable functional groups such as acrylate, methacrylate, acrylamide, methacrylamide, N-vinyl lactam, N-vinylamide, and styryl functional groups. Examples of silicone-containing components which are useful in this invention may be found in U.S. Patent Nos. 3,808,178; 4,120,570; 4,136,250; 4,139,513; 4,139,692; 4,153,641; 4,740,533; 5,034,461; 5,070,215; 5,260,000; 5,358,995; 5,760,100; 5,962,548; 5,998,498; 6,367,929; 6,849,671; 6,943,203; 7,052,131; 7,521,488; 7,825,170; and 7,939,579 and European Patent No. 080539.

Suitable silicone-containing components include compounds of Formula I

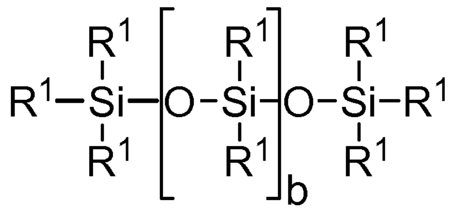

Formula I

wherein:

$R^1$ is independently selected from reactive groups, monovalent alkyl groups, or monovalent aryl groups, any of the foregoing which may further comprise functionality selected from hydroxy, amino, oxa, carboxy, alkyl carboxy, alkoxy, amido, carbamate, carbonate, halogen or combinations thereof; and monovalent siloxane chains comprising 1-100 Si-O repeat units which may further comprise functionality selected from alkyl, hydroxy, amino, oxa, carboxy, alkyl carboxy, alkoxy, amido, carbamate, halogen or combinations thereof;
where b = 0 to 500 (such as 0 to 100, such as 0 to 20), where it is understood that when b is other than 0, b is a distribution having a mode equal to a stated value; and
wherein at least one $R^1$ comprises a reactive group, and in some embodiments from one to three $R^1$ comprise reactive groups.

As used herein "reactive groups" are groups that can undergo free radical and/or cationic polymerization. Non-limiting examples of free radical reactive groups include (meth)acrylates, styryls, vinyls, vinyl ethers, $C_{1-6}$alkyl(meth)acrylates, (meth)acrylamides, $C_{1-6}$alkyl(meth)acrylamides, N-vinyllactams, N-vinylamides, $C_{2-12}$alkenyls, $C_{2-12}$alkenylphenyls, $C_{2-12}$alkenylnaphthyls, $C_{2-6}$alkenylphenyl$C_{1-6}$alkyls, O-vinylcarbamates and O-vinylcarbonates. Non-limiting examples of cationic reactive groups include vinyl ethers or epoxide groups and mixtures thereof. In one embodiment the free radical reactive groups comprises (meth)acrylate, acryloxy, (meth)acrylamide, and mixtures thereof.
Suitable monovalent alkyl and aryl groups include unsubstituted monovalent $C_1$ to $C_{16}$alkyl groups, $C_6$-$C_{14}$ aryl groups, such as substituted and unsubstituted methyl, ethyl, propyl, butyl, 2-hydroxypropyl, propoxypropyl, polyethyleneoxypropyl, combinations thereof and the like, or unsubstituted $C_{1-4}$ alkyl groups.
In one embodiment b is zero, one $R^1$ is a reactive group, and at least 3 $R^1$ are selected from monovalent alkyl groups having one to 16 carbon atoms, and in another embodiment from monovalent alkyl groups having one to 6 carbon atoms. Non-limiting examples of silicone components of this embodiment include (3-methacryloxy-2-hydroxypropoxy)propyl-bis(trimethylsiloxy)methylsilane ("SiGMA"; structure in Formula II),

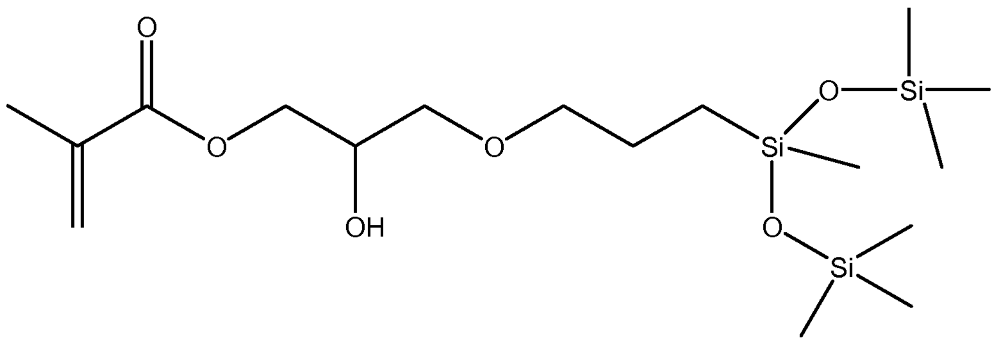

Formula II

2-hydroxy-3-methacryloxypropyloxypropyl-tris(trimethylsiloxy)silane, 3-methacryloxypropyltris(trimethylsiloxy)silane ("TRIS"), 3-methacryloxypropylbis(trimethylsiloxy)methylsilane, and 3-methacryloxypropylpentamethyl disiloxane.
In another embodiment, b is 2 to 20, 3 to 15 or 3 to 10; at least one terminal $R^1$ comprises a reactive group and the remaining $R^1$ are selected from monovalent alkyl groups having 1 to 16 carbon atoms, or monovalent alkyl groups having 1 to 6 carbon atoms. In yet another embodiment, b is 3 to 15, one terminal $R^1$ comprises a reactive group, the other terminal $R^1$ comprises a monovalent alkyl group having 1 to 6 carbon atoms and the remaining $R^1$ comprise monovalent alkyl group having 1 to 3 carbon atoms. Non-limiting examples of silicone components of this embodiment include (mono-(2-hydroxy-3-methacryloxypropyl)-propyl ether terminated polydimethylsiloxane (400-1000 MW)) ("OH-mPDMS"; structure in Formula III),

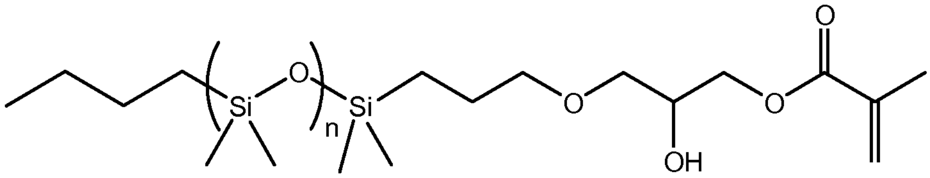

Formula III

monomethacryloxypropyl terminated mono-n-butyl terminated polydimethylsiloxanes (for example, with 800-1000 MW), ("mPDMS"; structure in Formula IV).

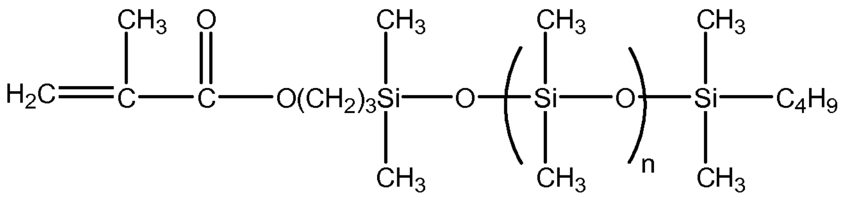

Formula IV

In another embodiment b is 5 to 400 or from 10 to 300, both terminal $R^1$ comprise reactive groups and the remaining $R^1$ are independently selected from monovalent alkyl groups having 1 to 18 carbon atoms which may have ether linkages between carbon atoms and may further comprise halogen.

In another embodiment, one to four $R^1$ comprises a vinyl carbonate or carbamate of Formula V:

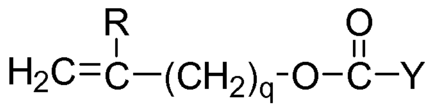

Formula V

wherein: Y denotes O-, S- or NH-; R denotes, hydrogen or methyl; and q is 0 or 1.

The silicone-containing vinyl carbonate or vinyl carbamate monomers specifically include: 1,3-bis[4-(vinyloxycarbonyloxy)but-1-yl]tetramethyl-disiloxane; 3-(vinyloxycarbonylthio) propyl-[tris (trimethylsiloxy)silane]; 3-[tris(trimethylsiloxy)silyl] propyl allyl carbamate; 3-[tris(trimethylsiloxy)silyl] propyl vinyl carbamate; trimethylsilylethyl vinyl carbonate; trimethylsilylmethyl vinyl carbonate, and the compound of Formula VI.

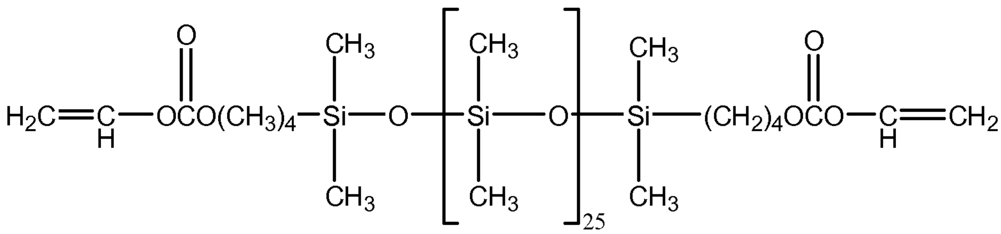

Formula VI

Where biomedical devices with modulus below about 200 are desired, only one $R^1$ shall comprise a reactive group and no more than two of the remaining $R^1$ groups will comprise monovalent siloxane groups.

Another suitable silicone containing macromer is compound of Formula VII (in which x + y is a number in the range of 10 to 30) formed by the reaction of fluoroether, hydroxy-terminated polydimethylsiloxane, isophorone diisocyanate and isocyanatoethylmethacrylate.

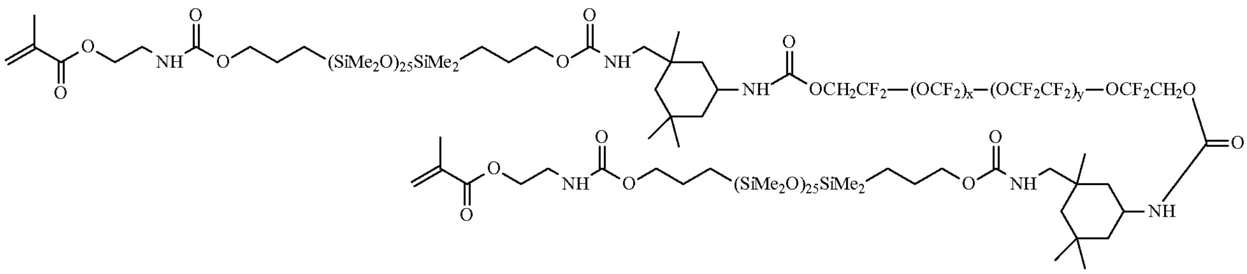

Formula VII

Other silicone components suitable for use in this invention include those described is WO 96/31792 such as macromers containing polysiloxane, polyalkylene ether, diisocyanate, polyfluorinated hydrocarbon, polyfluorinated ether and polysaccharide groups. Another class of suitable silicone-containing components includes silicone containing macromers made via GTP, such as those disclosed in U.S. PatNos. 5,314,960; 5,331,067; 5,244,981; 5,371,147; and 6,367,929. U.S. Pat. Nos. 5,321,108; 5,387,662 and 5,539,016 describe polysiloxanes with a polar fluorinated graft or side group having a hydrogen atom attached to a terminal difluoro-substituted carbon atom. US 2002/0016383 describe hydrophilic siloxanyl methacrylates containing ether and siloxanyl linkanges and crosslinkable monomers containing polyether and polysiloxanyl groups. Any of the foregoing polysiloxanes can also be used as the silicone-containing component in this invention. Other silicone-containing materials that may be used with this invention include acquafilcon A, balafilcon A, galyfilcon A, senofilcon A, comfilcon, lotrafilcon A, and lotrafilcon B.

Where a modulus of less than about 120 psi is desired, the majority of the mass fraction of the silicone-containing components used in the contact lens formulation should contain only one polymerizable functional group ("monofunctional silicone containing component"). In this embodiment, to insure the desired balance of oxygen transmissibility and modulus it is preferred that all components having more than one polymerizable functional group ("multifunctional components") make up no more than 10 mmol/100 g of the reactive components, and preferably no more than 7 mmol/100 g of the reactive components.

The silicone component may be selected from the group consisting of monomethacryloxypropyl terminated, mono-n-alkyl terminated polydialkylsiloxane; bis-3-acryloxy-2-hydroxypropyloxypropyl polydialkylsiloxane; methacryloxypropyl-terminated polydialkylsiloxanes; mono-(3-methacryloxy-2-hydroxypropyloxy)propyl terminated, mono-alkyl terminated polydialkylsiloxane; and mixtures thereof.

The silicone component may be selected from the group consisting of mono(meth)acryloxypropyl terminated, mono-n-alkyl terminated polydialkylsiloxane; bis-3-acryloxy-2-hydroxypropyloxypropyl polydialkylsiloxane; (meth)acryloxypropyl-terminated polydialkylsiloxane; mono-(3-(meth)acryloxy-2-hydroxypropyloxy)propyl terminated, mono-alkyl terminated polydialkylsiloxane; monomethacrylamidopropyl terminated, mono-n-alkyl terminated polydialkylsiloxane; bis-3-(meth)acrylamido-2-hydroxypropyloxypropyl polydialkylsiloxane; (meth)acrylamidopropyl-terminated polydialkylsiloxane; mono-(3-(meth)acrylamido-2-hydroxypropyloxy)propyl terminated, mono-alkyl terminated polydialkylsiloxane; and mixtures thereof.

The silicone component may be selected from monomethacrylate terminated polydimethylsiloxanes; bis-3-acryloxy-2-hydroxypropyloxypropyl polydialkylsiloxane; and mono-(3-methacryloxy-2-hydroxypropyloxy)propyl terminated, mono-butyl terminated polydialkylsiloxane; and mixtures thereof.

The silicone component may have an average molecular weight of from about 400 to about 4000 daltons.

The silicone containing component(s) may be present in amounts up to about 95 weight %, from about 10 and about 80 or from about 20 and about 70 weight %, based upon all reactive components of the reactive mixture (e.g., excluding diluents).

The reaction mixtures from which the silicone hydrogels of the present application are formed further comprise at least one hydrophilic component. The hydrophilic components are known in the art and impart water content and improved wettability (measured via contact angle) to the resulting hydrogels and ophthalmic devices, including contact lenses. Suitable hydrophilic components include known hydrophilic momomers used to prepare hydrogels. For example monomers containing acrylic groups ($CH_2$=CROX, where R is hydrogen or $C_{1-6}$ alkyl an X is O or N) or vinyl groups (C=$CH_2$) may be used. Examples of hydrophilic monomers are N,N dimethylacrylamide, 2-hydroxyethyl methacrylate, 2-hydroxypropyl methacrylate, glycerol methacrylate, 2-hydroxyethyl methacrylamide, 2-hydroxypropyl methacrylamide, polyethyleneglycol monomethacrylate, methacrylic acid, acrylic acid, N-vinyl pyrrolidone, N-vinyl-N-methyl acetamide, N-vinyl-N-ethyl acetamide, N-vinyl-N-ethyl formamide, N-vinyl formamide. Reactive and non-reactive polymers and copolymers thereof may also be used.

## Manufacture of Contact Lens

Contact lenses may be manufactured from a reactive mixture comprising a silicone component. The reactive mixture of the present invention may be cured via any known process for molding the reaction mixture in the production of contact lenses, including spincasting and static casting. Spincasting methods are disclosed in U.S. Patents Nos. 3,408,429 and 3,660,545, and static casting methods are disclosed in U.S. Patents Nos. 4,113,224 and 4,197,266. In one embodiment, the contact lenses of this invention are formed by the direct molding of the hydrogels, which is economical, and enables precise control over the final shape of the hydrated contact lens. For this method, the reaction mixture is placed in a mold having the shape of the final desired hydrogel and the reaction mixture is subjected to conditions whereby the monomers polymerize, to thereby produce a polymer in the approximate shape of the final desired product.

After curing, the contact lens may be subjected to extraction to remove unreacted components and release the contact lens from the contact lens mold. The extraction may be done using conventional extraction fluids, such organic solvents, such as alcohols or may be extracted using aqueous solutions.

Aqueous solutions are solutions which comprise water. In one embodiment the aqueous solutions comprise at least about 30 weight % water, at least about 50 weight % water, at least about 70% water or at least about 90 weight% water. Aqueous solutions may also include additional water soluble components such as release agents, wetting agents, slip agents, pharmaceutical and nutraceutical components, combinations thereof and the like.

Release agents are compounds or mixtures of compounds which, when combined with water, decrease the time required to release a contact lens from a mold, as compared to the time required to release such a contact lens using an aqueous solution that does not comprise the release agent.

Extraction can be accomplished, for example, via immersion of the contact lens in an aqueous solution or exposing the contact lens to a flow of an aqueous solution. Extraction can also include, for example, one or more of: heating the aqueous solution; stirring the aqueous solution; increasing the level of release aid in the aqueous solution to a level sufficient to cause release of the contact lens; mechanical or ultrasonic agitation of the contact lens; and incorporating at least one leach aid in the aqueous solution to a level sufficient to facilitate adequate removal of unreacted components from the contact lens. The foregoing may be conducted in batch or continuous processes, with or without the addition of heat, agitation or both.

Physical agitation may also be used to facilitate leach and release. For example, the contact lens mold part to which a contact lens is adhered, can be vibrated or caused to move back and forth within an aqueous solution. Other embodiments may include ultrasonic waves through the aqueous solution.

The contact lenses may be sterilized by known means such as, but not limited to autoclaving. Autoclaving is preferably conducted in the sealed packaging and packaging solution in which the contact lens will be sold. Buffered saline solutions, including phosphate and borate buffered saline solutions may be used. The buffer solutions are aqueous solutions comprising tonicity adjusting agents, buffering agents and may include non-ionic, non-polymerizable polymeric wetting agents including to poly-N-vinyl pyrrolidone, poly-N-vinyl-2- piperidone, poly-N-vinyl-2-caprolactam, poly-N-vinyl-3-methyl-2- caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2- pyrrolidone, and poly-N-vinyl-4,5-dimethyl-2-pyrrolidone, polyvinylimidazole, poly-N-N-dimethylacrylamide, polyacrylamide, polyhydroxypropyl(meth)acrylate, polyhydroxypropyl(meth)acrylamide, polyvinyl alcohol, polyethylene oxide, poly 2 ethyl oxazoline, phosphorylcholine, heparin polysaccharides and polysaccharides, mixtures and copolymers thereof and the like. Poly-N-vinylpyrrolidone, poly-N-N-dimethylacrylamide, polyacrylamide, polyhydroxypropyl(meth)acrylate, polyhydroxypropyl(meth)acrylamide are particularly preferred. The wetting agents may be included in amounts between about 10 ppm and about 5wt%, about 10 ppm and about 2000 ppm and about 100 to about 1000 ppm.

## Clay Treatment of Contact Lens Surface

As discussed above, one or more clays are applied to at least a portion of at least one surface of the contact lens. In one embodiment, the clay is an aluminum silicate. Examples of aluminum silicates include, but are not limited to: kaolins, such as kaolinite, dickite, halloysite, and nacrite; smectites, such as montmorillonites (e.g., bentonites, hectorites, Nanomer® clays such as Nanomer® PGV), pyrophyillites, talcs, vermiculites, saucites, nontronites, and saponites; illites; chlorites; sepiolites; zeolites; and attapulgites. Clays useful for this invention may be acidic, neutral or alkaline. The clay is preferably comprised of water insoluble particles, and is preferably comprised of particles less than 25 microns in diameter. Montmorillonites often have a sheet-type or plate structure. Although their dimensions in the length and width directions can be measured in the hundreds of nanometers, the mineral's thickness is often only about one nanometer. As a result, individual sheets can have aspect ratios (L/w) varying from 200 to 1000, with a majority of platelets in the 200-400 range after purification. A typical molecular formula for montmorillonite is, for example,

$(Na,Ca)_{0.33}(Al,Mg)_2(Si_4O_{10})(OH)_2 \cdot nH_2O$

In one embodiment, the clay is adhered directly to the surface of the contact lens, and in one embodiment a contact lens which has not been previously coated. While not wishing to be bound by theory, the clay may be adhered to the surface of the contact lens by one or more boding mechanism, such as hydrogen bonding, or by formation of siloxane bonds, which may for example be formed by reaction of $Si\text{-}O^-$ groups with $Si\text{-}O^-$ Si groups on the surface. It is a benefit of the present invention that no pretreatment step is required. Pretreatment steps that can be foregone include coating, contacting or treating the lens with a positively charged polyelectrolyte, treatment of the lens with a separate coupling agent, including a cationic component in the reactive mixture from which the contact lens is made and the like.

The at least one clay is mixed with a solution to form a suspension. The solution may be aqueous based or may be any liquid used in the manufacture of contact lenses. In one embodiment the solution is an aqueous solution. The pH of the solution is not critical, and deionized water may be used with or without buffer. Thus in one embodiment the solution is deionized water, and in another is a buffered aqueous solution. Suitable buffers include borate and phosphate buffers.

The clays may be included in the solution in amounts between about 0.001% and about 10 wt%, about 0.01 and about 10 wt% and between about 0.01 and about 5 wt%. In embodiments where the suspensions are not stable, the suspensions are stirred or agitated prior to and during contacting with the contact lenses.

Suitable treatment temperatures include those between the freezing and boiling points of the selected solvent, and convenient temperatures can range from about 10 to about 100°C and from about ambient temperature to about 100°C.

Suitable contacting means include spraying, dipping, wiping, rolling, combinations thereof and the like. The entire lens may be coated, one surface of the lens may be coated or only a portion of a surface may be coated. For example, when the biomedical device is a contact lens, the entire lens may be coated, only one side of the lens may be coated (for example either the back curve which rests on the cornea and conjuctiva, or the front surface which is in contact with the eyelids and air), or only a portion of a surface may be coated (for example a portion of either surface which covers the iris, pupil or conjuctiva).

The contact time used will be a length of time sufficient to coat the surface to the extent desired. Desirable reductions in contact angle may be achieved in relatively short periods of time, such as less than about 1 hour, less than about 30 minutes, less than about 10 minutes, and in some embodiments less than about 5 minutes. Contact times longer than those disclosed herein are less desirable as thicker clay coatings may inhibit permeability of desirable components, such as oxygen, through the lens. Thus, in one embodiment the contact time and a clay concentration are selected to retain about 90% of the oxygen permeability of the untreated contact lens. It should be appreciated that the concentration of the clay in the solution, reaction temperature and time are all related, and that higher concentrations of clay and/or increased temperatures may allow for shorter contacting times.

<u>XPS Method</u>

Each contact lens sample is washed in ultra-pure water in a following manner before being mounted on the special dome shaped sample mount: (1) A quick rinse; (2) A 10min soak in a new supply of water; and (3) A second quick rinse in a new supply of water. For the hydrated state cold stage analysis, a droplet of deionized ("DI") water is placed near the center of each sample prior to freezing. Then the samples are frozen (using liquid nitrogen) in the intro chamber before the initial pumpdown. The ice is sublimated while pumping down the intro chamber. This process preserves the hydrated state of the surface. Once the ice is sublimated, the samples are introduced into the analytical chamber of the instrument. The spectral acquisitions are performed while the sample stage is constantly cooled by liquid nitrogen.

<u>Analytical Parameters</u>

| | |
|---|---|
| Instrument | PHI 5802 Multitechnique |
| X-ray source | Monochromatic Al $K_\alpha$ 1486.6eV |
| Acceptance Angle | ±23° |
| Take-off angles | 45° |
| Analysis area | 800μm - surface |
| Charge Correction | C-C,H in Cls set to 284.8 eV |
| Charge Compensation | Electron and Ion floods |

(continued)

| | |
|---|---|
| Cold Stage Sample Temperature: | -50°...-100°C |

Sessile Drop Contact Angle Method

Surface wettability of contact lenses can be determined using a sessile drop contact angle technique using KRUSS DSA-100™ instrument at room temperature and using DI water as probe liquid. The contact lenses to be tested (5 per lot) are soaked in borate buffered surfactant free packing solution to remove carry over from original contact lens packing solution. Each test contact lens is placed on a conforming contact lens holder with the front curve facing outwards and blotted on Whatman #1 filter paper for 20 seconds. Immediately after blotting, the contact lens together with the contact lens holder is placed in the sessile drop instrument sample stage, ensuring proper centering of needle to deliver the water droplet. A 3 microliter of DI water droplet is generated using DSA 100-Drop Shape Analysis software ensuring that the liquid drop is hanging away from the contact lens. The droplet is made in contact with the contact lens surface by raising the stage upwards. The liquid droplet is allowed to equilibrate on the contact lens surface for 1-3 seconds and the contact angle is determined using the built-in analysis software. The reduction in values for contact angle indicate that surface of the contact lens is more wettable.

Lipid Uptake Analysis

A standard curve is set up for each contact lens type under investigation. Tagged cholesterol (cholesterol labeled with NBD ([7-nitrobenz-2-oxa-1,3-diazol-4-yl], CH-NBD; Avanti, Alabaster, AL)) is solubilized in a stock solution of 1 mg /mL lipid in methanol at 35°C. Aliquots are taken from this stock to make standard curves in phosphate-buffered saline (PBS) at pH 7.4 in a concentration range from 0 to 100 microgram /mL.

One milliliter of standard at each concentration is placed in the well of a 24-well cell culture plate. 10 contact lenses of each type are placed in another 24-well plate and soaked alongside the standard curve samples in 1 mL of a concentration of 20 microgram /ml of CH-NBD. Another set of contact lenses (5 contact lenses) are soaked in PBS without lipids to correct for any autofluorescence produced by the contact lens itself. All concentrations are made up in phosphate buffered saline (PBS) at pH 7.4. Standard curves, test plates (containing contact lenses soaked in CH-NBD) and control plates (containing contact lenses soaked in PBS) are all wrapped in aluminum foil to maintain darkness and are incubated for 24 hours, with agitation at 35.C. After 24 hours the standard curve, test plates and control plates are removed from the incubator. The standard curve plates are immediately read on a micro-plate fluorescence reader (Synergy HT).

The contact lenses from the test and control plates are rinsed by dipping each individual contact lens 3 to 5 times in 3 consecutive vials containing approximately 100 ml of PBS to ensure that only bound lipid would be determined without lipids carryover. The contact lenses are then placed in a fresh 24-well plate containing 1 mL of PBS in each well and read on the fluorescence reader. After the test samples are read, the PBS is removed, and 1 mL of a fresh solution of CH-NBD are placed on the contact lenses in the same concentrations as previously mentioned and placed back in the incubator at 35°C, with rocking, until the next period. This procedure is repeated for 15 days until complete saturation of lipids on contact lenses. Only the lipid amount obtained at saturation is reported.

PQ-1 Uptake Analysis

The uptake of polyquaternium-1 (PQ-1) has been indicated as a possible source irritation with contact lenses. Uptake is calculated as the difference of the PQ-1 preservative content in the test solution before the contact lenses are immersed and the concentration in the test solution after 72 hours. Contact lenses are placed into polypropylene contact lens cases (one lens per 3 mL) with Optifree Replenish (which contains 0.001 wt% PQ-1, 0.56% citrate dihydrate and 0.021 % citric acid monohydrate (wt/wt) and is commercially available from Alcon). A control lens case, containing 3 mL of solution, but no contact lens is also prepared. The contact lenses and control solutions are allowed to sit at room temperature for 72 hours. 1 ml of solution is removed from each of the samples and controls and mixed with trifluoroacetic acid (10 $\mu$L). The analysis is conducted using HPLC/ELSD and a Phenomenex Luna C5 (4.6 mm x 50 mm; 5 $\mu$m particle size) column and the following conditions:

Instrument: Agilent 1200 HPLC with an ELSD (or equivalent)
ELSD: T = 100°C, Gain = 12, Pressure = 4.4 bar, Filter = 3s (Note: ELSD parameters may change from instrument to instrument)
HPLC Column: Phenomenex Luna C5 (4.6 mm x 50 mm; 5 $\mu$m particle size)
Mobile Phase A: $H_20$ (0.1% TFA)

Mobile Phase B: Acetonitrile (0.1% TFA)
Column Temperature: 40 °C
Injection Volume: 100 μL
HPLC Run Conditions (Table A):

Table A

| Time (minutes) | %A | %B | Flow Rate (mL/min) |
|---|---|---|---|
| 0.00 | 100 | 0 | 1.2 |
| 1.00 | 100 | 0 | 1.2 |
| 5.00 | 0 | 100 | 1.2 |
| 8.50 | 0 | 100 | 1.2 |
| 8.60 | 100 | 0 | 1.2 |
| 11.00 | 100 | 0 | 1.2 |

Standard Preparation

Alcon Opti-Free Replenish is used as the stock solution (PQ-1 concentration = 10 mcg/mL). A series of analytical standards is prepared as described below. They are diluted to volume with multi-purpose contact lens solution prepared without PQ-1 and mixed well (see Table C).

Working Standards Preparation from Opti-Free Replenish (Table B)

Table B

| Working Standard Name | Volume of Opti-Free Replenish (mL) | Volume of Diluent (mL) | Final Volume (mL) | Approximate PQ-1 Concentration (μg/mL) |
|---|---|---|---|---|
| Std A | 0.2 | 0.8 | 1.0 | 2.0 |
| StdB | 0.4 | 0.6 | 1.0 | 4.0 |
| Std C | 0.6 | 0.4 | 1.0 | 6.0 |
| Std D | 0.8 | 0.2 | 1.0 | 8.0 |
| Std E | 1.0 | 0.0 | 1.0 | 10.0 |
| Note: Working standards are prepared directly in autosampler vials. | | | | |

Table C

| INGREDIENT | WEIGHT (grams) |
|---|---|
| PVP (K90) | 1.50 |
| Poloxamer F-127 | 4.5 |
| Sodium Chloride | 5.5 |
| Potassium Phosphate Monobasic (g) | 1.44 |
| Disodium Hydrogen Phosphate, Dihydrate (g) | 2.57 |
| Diethylene Triamine Pentaacetic acid (g) | 0.40 |
| Calcium Hydroxide (g) | 0.075 |
| Sodium Citrate (g) | 6.5 |
| Sodium Chlorite (80%) | 0.625 |
| Hydrogen Peroxide (30%) | 0.70 |
| Water | 1000.00 |

Sample/Standard Preparation for Analysis

1 milliliter of MPS sample (or standard) and 10 microliters of trifluoroacetic acid is placed into an autosampler vial which is capped and shaken well.

Analysis

1. Six injections of "StdD" are performed to evaluate system suitability. The RSD% of the peak areas and retention times must be $\leq$ 5% to pass system suitability.
2. Working standards A-E are injected to create a calibration curve. The square of the correlation coefficient ($r^2$) must be $\geq$ 0.99.
3. Samples are injected followed by a bracketing standard (StdD). The peak area of the bracketing standard must be $\pm$ 10% of the averaged peak areas from the system suitability injections.

Calculations

An absorbance vs. concentration graph is constructed by plotting the peak area value that corresponds to the concentration of each PQ-1 standard solution. The concentration of PQ-1 in sample is calculated by solving a quadratic equation. This calculation should be performed by Chemstation or Empower software.

$$Y = ax^2 + bx + c$$

Y= Peak area
X = concentration of PQ-1 in prepared sample
A and B = equation constants
C = y-intercept

<u>Lysozyme Uptake Analysis</u>

Lysozyme is a naturally-occurring antibacterial protein. Uptake is measured as follows: The lysozyme solution used for the lysozyme uptake testing contained lysozyme from chicken egg white (Sigma, L7651) solubilized at a concentration of 2 mg/ml in phosphate saline buffer supplemented by sodium bicarbonate at 1.37g/l and D-Glucose at 0.1 g/l. Three contact lenses for each example are tested using each protein solution, and three are tested using PBS as a control solution. The test contact lenses are blotted on sterile gauze to remove packing solution and aseptically transferred, using sterile forceps, into sterile, 24 well cell culture plates (one lens per well) each well containing 2 ml of lysozyme solution. Each contact lens is fully immersed in the solution. 2 ml of the lysozyme solution is placed in a well without a contact lens as a control.

The plates containing the contact lenses and the control plates containing only protein solution and the contact lenses in the PBS, are parafilmed to prevent evaporation and dehydration, placed onto an orbital shaker and incubated at 35°C, with agitation at 100 rpm for 72 hours. After the 72 hour incubation period the contact lenses are rinsed 3 to 5 times by dipping contact lenses into three (3) separate vials containing approximately 200 ml volume of PBS. The contact lenses are blotted on a paper towel to remove excess PBS solution and transferred into sterile conical tubes (1 lens per tube), each tube containing a volume of PBS determined based upon an estimate of lysozyme uptake expected based upon on each contact lens composition. The lysozyme concentration in each tube to be tested needs to be within the albumin standards range as described by the manufacturer (0.05 microgram to 30 micrograms). Samples known to uptake a level of lysozyme lower than 100 $\mu$g per contact lens are diluted 5 times. Samples known to uptake levels of lysozyme higher than 500 $\mu$g per contact lens (such as etafilcon A contact lenses) are diluted 20 times. 1 ml aliquot of PBS is used for all samples other than etafilcon. 20ml are used for etafilcon A contact lens. Each control contact lens is identically processed, except that the well plates contained PBS instead of lysozyme solution.

Lysozyme uptake is determined using on-lens bicinchoninic acid method using QP-BCA kit (Sigma, QP-BCA) following the procedure described by the manufacturer (the standards prep is described in the kit) and is calculated by subtracting the optical density measured on PBS soaked contact lenses (background) from the optical density determined

on contact lenses soaked in lysozyme solution. Optical density is measured using a SynergyII Micro-plate reader capable for reading optical density at 562nm.

Water Content

The water content of contact lenses is measured as follows: Three sets of three contact lenses are allowed to sit in packing solution for 24 hours. Each contact lens is blotted with damp wipes and weighed. The contact lenses are dried at 60°C for four hours at a pressure of 0.4 inches Hg or less. The dried contact lenses are weighed. The water content is calculated as follows:

$$\% \text{ water content} = (\text{wet weight} - \text{dry weight}) / \text{wet weight} \times 100$$

The average and standard deviation of the water content are calculated for the samples and are reported.

Modulus, Tensile Strength and Elongation at Break

Tensile properties of a material are measured by using of a constant rate of movement type tensile testing machine equipped with a suitable load cell that is lowered to the initial gauge height. A suitable testing machine includes an Instron model 1122 or 5542. A dog-bone shaped sample having a 0.522 inch length, 0.276 inch "ear" width and 0.213 inch "neck" width is loaded into the grips and elongated at a constant rate of strain of 2 in/min. until it breaks. The initial gauge length of the sample (Lo) and sample length at break (Lf) are measured. Twelve specimens of each composition are measured and the average is reported. Percent elongation is = [(Lf - Lo)/Lo]x 100. Tensile modulus is measured at the initial linear portion of the stress/strain curve. The toughness is measured lb./$in^3$.

Oxygen Permeability (Dk)

The Dk is measured as follows. Contact lenses are positioned on a polarographic oxygen sensor consisting of a 4 mm diameter gold cathode and a silver ring anode then covered on the upper side with a mesh support. The radius of the sensor is 7.8 mm. The contact lens is exposed to an atmosphere of humidified 2.1% $O_2$ and the partial pressure of the cell constant reflects the lower oxygen concentration. The oxygen that diffuses through the contact lens is measured by the sensor. Contact lenses are either stacked on top of each other to increase the thickness or a thicker contact lens is used. The L/Dk of 4 samples with significantly different thickness values are measured and plotted against the thickness. The inverse of the regressed slope is the preliminary Dk of the sample. If the preliminary Dk of the sample is less than 90 barrer, then an edge correction of (1 + (5.88(CT in cm))) is applied to the preliminary L/Dk values. If the preliminary Dk of the sample is greater than 90 barrer, then an edge correction of (1 + (3.56(CT in cm))) is applied to the preliminary L/Dk values. The edge corrected L/Dk of the 4 samples are plotted against the thickness. The inverse of the regressed slope is the Dk of the sample. The reference values are those measured on commercially available contact lenses using this method. Balafilcon A contact lenses available from Bausch & Lomb give a measurement of approx. 79 barrer. Etafilcon contact lenses give a measurement of 20 to 25 barrer. (1 barrer = $10^{-10}$ ($cm^3$ of gas x $cm^2$)/($cm^3$ of polymer x sec x cm Hg)).

Haze Measurement

Haze is measured by placing a hydrated test lens in borate buffered saline in a clear 20 x 40 x 10 mm glass cell at ambient temperature above a flat black background, illuminating from below with a fiber optic lamp (Dolan-Jenner PL-900 fiber optic light with 0.5" diameter light guide set at a power setting of 4-5.4) at an angle 66° normal to the lens cell, and capturing an image of the lens from above, normal to the lens cell with a video camera (DVC 1300C:19130 RGB camera with Navitar TV Zoom 7000 zoom lens) placed 14 cm above the lens platform. The value of the background scatter (BS) is measured using a saline filled glass cell which is captured using EPIX XCAP V 2.2 software. The subtracted scattered light image is quantitatively analyzed, by integrating over the central 10 mm of the lens, and then comparing to a -1.00 diopter CSI Thin Lens®, which is arbitrarily set at a "CSI haze value" of 100, with no lens set as a haze value of 0. Five lenses are analyzed and the results are averaged to generate a haze value as a percentage of the standard CSI lens.

In the event that -1.00 diopter CSI Thin Lenses® are not available as a standard, a series of aqueous dispersions of stock latex spheres (commercially available as 0.49 μm Polystyene Latex Spheres - Certified Nanosphere Size Standards from Ted Pella, Inc., Product Number 610-30, or equivalent) can be used as standards. So for example, a

series of calibration samples were prepared in deionized water. Each solution of varying concentration was placed in a cuvette (2mm path length) and the solution haze was measured using the above method as reported in Table D.

Table D

| Solution | Concentration (wt% x $10^{-4}$) | Mean GS |
|---|---|---|
| 1 | 10.0 | 533 |
| 2 | 6.9 | 439 |
| 3 | 5.0 | 379 |
| 4 | 4.0 | 229 |
| 5 | 2.0 | 172 |
| 6 | 0.7 | 138 |

A suitable corrective factor can be derived by dividing the slope of the plot of Mean GS against the concentration (47.1) by the slope of an experimentally obtained standard curve, and multiplying this ratio times measured scatter values for lenses to obtain GS values.

"CSI haze value" may be calculated as follows:

$$\text{CSI haze value} = 100x(GS-BS)/(217-BS)$$

Where GS is gray scale and BS is background scatter.

Examples

These examples do not limit the invention. They are meant only to suggest a method of practicing the invention. Those knowledgeable in contact lenses as well as other specialties may find other methods of practicing the invention. The following abbreviations are used in the examples below:

| | |
|---|---|
| SiGMA | bis(trimethylsiloxy)methylsilylpropylglycerol methacrylate |
| DMA | N,N-dimethylacrylamide |
| HEMA | 2-hydroxyethyl methacrylate |
| Norbloc | 2-(2'-hydroxy-5-methacrylyloxyethylphenyl)-2H-benzotriazole |
| Darocur 1173 | 2-hydroxy-2-methylpropiophenone |
| PVP K-90 | poly(N-vinyl pyrrolidone) (K value 90) |
| TEGDMA | tetraethyleneglycol dimethacrylate |
| TRIS | 3-methacryloxypropyltris(trimethylsiloxy)silane |
| PQ-1 | Polyquaternium 1, or ethanol, 2,2',2'' -nitrilotris-, polymer with 1,4-dichloro-2-butene and N,N,N',N'-tetramethyl-2-butene-1,4-diamine |
| OH-mPDMS | Prepared as described in U.S. Patent Application 2006/0229423 |
| F-24 | acid-leached Bentonite with 1-3% crystalline silica |
| CGI 1850 | 1:1 (wgt) blend of 1-hydroxycyclohexyl phenyl ketone and bis(2,6-dimethoxybenzoyl)-2,4-4-trimethylpentyl phosphine oxide D3O |
| IPA | isopropyl alcohol |
| D3O | 3,7-dimethyl-3-octanol |

Example 1

A blend was made of 50% (wt) TRIS (Gelest SIM6487.6-06), 42% DMA, 8.0% HEMA, and 0.3% Darocur 1173. The blend was allowed to sit in a nitrogen atmosphere for about 30 minutes, then was placed, while under a nitrogen atmosphere, into plastic contact lens molds and irradiated for 30 minutes using Philips TL20W/09N UV fluorescent bulbs. The mold halves were separated, and dry lenses were removed by flexing the mold half containing the lens. Twelve of these lenses were placed into 28 x 5 mm plastic tissue capsules (from Simport Scientific, (Beloeil, Quebec, Canada) which were placed into deionized water and allowed to hydrate for about 30 minutes. Six hydrated lenses in plastic cells were placed into a rapidly stirring suspension of 20 grams of a granular combination of 97-99% acid-leached Bentonite with 1-3% crystalline silica (combination sold as Grade F-24 from BASF, Raritan, NJ) in 200 ml deionized water heated to 90-95°C. After 15 minutes, the cells and lenses were removed from the suspension. The lenses were removed from

the cells and rinsed several times with deionized water. They were placed individually into glass vials with borate buffered saline.

The diameters of three treated lenses and three untreated lenses were measured in the X and Y direction. The results in Table 1 suggest that the bulk properties of the lenses were unaffected.

The sessile drop contact angle of six treated and six untreated lenses were then determined. The results in Table 1 show that the wettability of the lenses was dramatically improved in the treated lens.

Table 1

| | Diameter | Contact angle |
|---|---|---|
| Untreated controls | 16.72 ± 0.09 mm | 112 ± 8.5° |
| Treated lenses | 16.63 ± 0.09 mm | 28.1 ± 4.3° |

Example 2

Silicone hydrogel lenses made from senofilcon A, and sold under the name ACUVUE® OASYS® Brand Contact Lenses with Hydraclear® Plus (Johnson & Johnson Vision Care, Inc., Jacksonville, FL), were washed several times with deionized water, placed into tissue capsules, and placed into a suspension of 9 (wt) % F-24 in deionized water, which was heated to 90-100°C and stirred vigorously for about 20 minutes. The lenses were rinsed in borate buffered saline, with a digital rub to remove a residual film of nanoclay on their surfaces. The sessile drop contact angle and the diameter of the lenses were determined and are shown in Table 2.

Table 2

| | Diameter | Contact angle |
|---|---|---|
| Untreated controls | 13.93 ± 0.07 mm | 52 ± 8.3° |
| Treated lenses | 13.95 ± 0.03 mm | 33.1 ± 6.2° |

Example 3

Twenty ACUVUE® OASYS® Brand Contact Lenses with Hydraclear® Plus were removed from their packages and rinsed three times in deionized water. They were placed into tissue capsules. Lenses were stirred for varying lengths of time in a 3% (wt) dispersion of F-24 in deionized water at 90-100°C. The lenses were rinsed in borate buffered saline, with a digital rub to remove residual nanoclay. They were then autoclaved. Their sessile drop contact angles were measured and are reported in Table 3.

Table 3

| Treatment time | Contact angle |
|---|---|
| 0 minutes | 50.7 ± 3.1 |
| 1 minute | 21.5 ± 3.7 |
| 3 minutes | 20.1 ± 2.5 |
| 6 minutes | 20.7 ± 5.6 |

Examples 4 and 5

The process of Example 3 was repeated using other nanoclays in place on F-24 and using a treatment time of 3 minutes. The results are shown in Table 4.

Table 4

| Example | Nanoclay | Vendor | Contact angle |
|---|---|---|---|
| Control | None | - | Ranged from 49 to 63° |
| 4 | Zeolite type: ZSM-5 | ACROS Organics [Geel, Belgium] | 19.6 ± 5.9° |

(continued)

| Example | Nanoclay | Vendor | Contact angle |
|---|---|---|---|
| 5 | Activated Bentonite Nanoclay, Grade F-20 | BASF | 17.2 ± 2.6° |

Examples 6 and 7

ACUVUE® OASYS® Brand Contact Lenses with Hydraclear® Plus were treated with F-24 or Zeolite ZSM-5 following the procedure of Example 4. The lenses were tested for a number of properties. The results are shown in Table 5.

Table 5

| | Untreated lens | Ex. 6 - F-24 treated | Ex. 7 - Zeolite ZSM-5 treated |
|---|---|---|---|
| Sessile drop contact angle | 50.9 ± 6.0° | 14.8 ± 2.8° | *18.6 ± 3.2° |
| Diameter | 13.93 ± 0.08 mm | 13.9 ± 0.1 mm | 13.87 ± 0.02 mm |
| Modulus | 90 ± 13 psi | 92 ± 9 psi | 96 ± 12 psi |
| Elongation at break | 241 ± 59% | 188 ± 84% | 129 ± 61% |
| Water content | 39.8 ± 0.2% | 39.9 ± 0.2% | 39.6 ± 0.1% |
| DK | 110 barrers | 112 barrers | 114 barrers |
| Lipid uptake | 30.0 ± 2.6 μg | 19.9 ± 2.2 μg | 18.4 ± 4.2 μg |
| Lysozyme uptake | 5.7 ± 0.3 μg | 12.3 ± 2.7 μg | 16.2 ± 0.8 μg |
| PQ-1 uptake | 1.0 ± 2.1% | 6.1 ± 3.6% | 2.0 ± 0.6% |
| *Lenses were tested within 24 hours after treatment. After several days the contact angle of these lenses had increased to 35-45°. | | | |

Example 8

Fifteen ACUVUE® OASYS® Brand Contact Lenses with Hydraclear® Plus were removed from their packages and rinsed three times in deionized water. They were placed into tissue capsules. Sets of five lenses were stirred for 3 minutes in a 3% (wt) dispersion of F-24 in deionized water at varying temperatures as shown in Table 6. The lenses were rinsed in borate buffered saline, with a digital rub to remove residual film. They were then autoclaved. The sessile drop contact angles were measured and are shown in Table 6.

Table 6

| Temperature | Contact angle |
|---|---|
| 30-40°C | 19.0 ± 1.8° |
| 50-60 °C | 17.8 ± 4.2° |
| 70-80°C | 19.5 ± 4.9° |

Example 9

Fifteen ACUVUE® OASYS® Brand Contact Lenses with Hydraclear® Plus were removed from their packages and rinsed three times in deionized water. They were placed into tissue capsules. Sets of five lenses were stirred for 3 minutes in a varied concentrations of F-24 in a dispersion in deionized water at 90-100° as shown in Table 7. The lenses were rinsed in borate buffered saline, with a digital rub to remove residual film. They were then autoclaved. The sessile drop contact angles were measured and are shown in Table 7.

Table 7

| Temperature | Contact angle |
|---|---|
| 1.0% | 24.5 ± 11.5° |
| 0.5% | 22.8 ± 4.2° |
| 0.25% | 23.9 ± 6.6° |
| 0.1% | 21.6 ± 4.1° |
| 0.01% | 27.5 ± 5.3° |
| Untreated control | 50.4 ± 3.4° |

Example 10

Several ACUVUE® OASYS® Brand Contact Lenses with Hydraclear® Plus were removed from their packages and rinsed three times in deionized water. They were placed into tissue capsules. They were stirred for 3 minutes in a 3% (wt) dispersion of F-24 in deionized water at 90-100°. The lenses were rinsed in borate buffered saline, but were not digitally rubbed to remove residual film. One lens was placed into a glass vial with 5mm glass beads, and filled to the top of the beads with borate buffered saline. The vial was vortexed for about a minute three times with replacement of the saline each time. The lens appeared to be free of residual film.

Comparative Example 10a

Five ACUVUE® OASYS® Brand Contact Lenses with Hydraclear® Plus were removed from their packages and rinsed three times in deionized water. They were placed into tissue capsules. They were stirred for 3 minutes in a 3% (wt) dispersion of silica gel (Aldrich Chemicals 200-400 mesh, 60Å) in deionized water at 90-100°. The lenses were rinsed in borate buffered saline, but they required several digital rubs, rinsing with borate buffered saline, to remove residual film. They were then autoclaved. The sessile drop contact angle of these lenses was 85.5 ± 3.4°.

Example 11

A blend was made with 4.33 parts (wt) macromer from Example 90 of US 7,691,916, 1.87 parts anhydrous ethanol, 1.73 parts TRIS, 2.6 parts DMA and 0.05 parts 2-hydroxy-2-methylpropiophenone. The blend was degassed by storing it in a nitrogen environment. Lenses were formed by curing in polypropylene molds about 5 inches under a Philips TL 20W/09N fluorescent UV bulbs for 30 minutes. Lenses were released into 95% ethanol. After about 2, hours the ethanol was replaced with borate buffered saline.

Five lenses were rinsed with deionized water, and placed into tissue capsules. They were stirred for 3 minutes in a 3% (wt) suspension of F-24 in deionized water with stirring at 90-100°. The lenses were rinsed in borate buffered saline, and then rinsed with digital rubbing to remove residual film. They were then autoclaved. The sessile drop contact angle of these lenses was 48.5 ± 36.2°, compared to 109.2 ± 2.1° for untreated lenses.

Example 12

Five soft plasma treated silicone hydrogel lenses made from balafilcon A, sold under the trade name Purevision (Bausch & Lomb, Rochester, NY) were rinsed with deionized water and placed into tissue capsules. They were stirred for 3 minutes in a 3% (wt) suspension of F-24 in deionized water with stirring at 90-100°. The lenses were then rinsed in borate buffered saline, and rinsed with digital rubbing to remove residual film. They were then autoclaved. The sessile drop contact angle of these lenses was 51.7 ± 6.3°, compared to 78.3 ± 5.6° for untreated lenses.

Example 13

Five ACUVUE® OASYS® Brand Contact Lenses with Hydraclear® Plus were removed from their packages and rinsed three times in deionized water. They were placed into tissue capsules. They were stirred for 3 minutes in a 0.1 % (wt) dispersion of F-24 in deionized water at room temperature (24°C). The lenses were then rinsed in borate buffered saline, with a digital rub to remove residual film, and then autoclaved. The sessile drop contact angle was 24.6 ± 4.2°.

Example 14

Following the general procedure of Example 3, ACUVUE® OASYS® Brand Contact Lenses with Hydraclear® Plus were treated with hydrophilic Bentonite nanoclay (Cat. No. 682659 from Aldrich Chemistry). After rinsing in borate buffered saline, the lenses were free of any visible residual surface film, and thus did not require a digital rub. The sessile drop contact angle and other lens properties were determined and are shown in Table 8.

Example 15

Following the procedure of Example 4, five ACUVUE® OASYS® Brand Contact Lenses with Hydraclear® Plus were treated with powdered Bentonite (Cat. No. 285234 from Aldrich Chemistry). After rinsing in borate buffered saline, the lenses were free of any visible residual surface film, and thus did not require a digital rub. The sessile drop contact angle of the treated lenses was 19.1 ± 3.1°, compared to 56.2 ± 2.0° for an untreated control lens.

Comparative Example 15a

0.80 g of sodium silicate solution (Sigma-Aldrich Cat.# 338443, 10.6% $Na_2O$, 26.5% $SiO_2$ in water) was added to 9.22 g borate buffered saline solution. Five ACUVUE® OASYS® Brand Contact Lenses with Hydraclear® Plus were washed several times with deionized water, placed into this sodium silicate solution, and shaken for 3 minutes. The lenses were left in this solution for about 62 hours at room temperature. During this time, the solution became gel-like and opaque. The lenses were removed from the opaque solution and rinsed with borate buffered saline solution. They were then autoclaved in borate buffered saline. Their sessile drop contact angles were then determined to be 71.0 ± 3.1°, compared to 56.2 ± 2.0° for an untreated control lens.

Example 16

Lenses were made by placing a blend of 28 parts (wt) SiGMA, 31 parts DMA, 31 parts OH-mPDMS, 6 parts HEMA, 2 parts Norbloc, 1.5 parts TEGDMA, 0.5 parts CGI 1850 and 30 parts D3O into plastic contact lens molds ad curing for 30 minutes using Philips TL 20W/03T fluorescent bulbs at room temperature. The molds were opened and lenses released into 70% (vol) IPA in 30% water. The lenses were extracted in IPA, then placed into borate buffered saline.

The lenses were washed with deionized water, and placed into a stirring suspension of 3% (wt) F-24 in deionized water at 90-100°C for 3 minutes. After treatment, the lenses were cleaned with a digital rub to remove a surface film. They were then autoclaved and their sessile drop contact angle was determined. The treated lenses had a contact angle of 101.2 ± 7.3°, compared to 106.1 ± 4.8° for an untreated control lens.

Example 17

Sixty ACUVUE® OASYS® Brand Contact Lenses with Hydraclear® Plus were removed from their packages and rinsed three times in deionized water. They were not placed into tissue capsules. Lenses were stirred in a 1% (wt) dispersion of powdered Bentonite (Cat. No. 285234 from Aldrich Chemistry) in deionized water for 10 minutes at room temperature. After rinsing in borate buffered saline the lenses were free of any visible residual surface film, and thus did not require a digital rub. The sessile drop contact angle and other lens properties were determined and are shown in Table 8. The results of XPS Method are shown in Table 9.

Table 8

| | Untreated lens | Ex. 14 - 3% Bentonite | Ex. 17 - 1% Bentonite |
|---|---|---|---|
| Sessile drop contact angle | 56.2 ± 2.0° | 15.8 ± 2.0° | 27.9 ± 3.1° |
| Diameter | 13.95 ± 0.03 mm | 13.96 ± 0.08 mm | 13.94 ± 0.02 mm |
| Modulus | 106 ± 13 psi | 106 ± 15 psi | 106 ± 8 psi |
| Elongation at break | 184 ± 58% | 181 ± 64% | 144 ± 66% |
| Water content | 40 ± 0% | 40 ± 0% | 40 ± 0% |
| Dk | 121 barrers | 118 barrers | 114 barrers |
| Lipid uptake | 29.7 ± 2.5 μg | 20.3 ± 2.3 μg | 25.0 ± 1.2 μg |

(continued)

| | Untreated lens | Ex. 14 - 3% Bentonite | Ex. 17 - 1% Bentonite |
|---|---|---|---|
| Lysozyme uptake | 5.2 ± 0.04 μg | 13.9 ± 1.6 μg | 10.4 ± 1.0 μg |
| PQ-1 uptake | ± % | ± % | ± % |
| Haze | 11 ± 0 | 50 ± 10 | 43 ± 7 |

Table 9

| Sample | C | N | O | Si | Na | Mg | Al | Ca | Fe |
|---|---|---|---|---|---|---|---|---|---|
| Example 17, untreated | 64.9 ± 0.3 | 3.5 ± 0 | 21.4 ± 0.0 | 10.1 ± 0.3 | 0.1 ± 0.1 | 0 | 0 | 0 | 0 |
| Example 17, treated 3 min. with F-24 | 53.6 ± 0.9 | 2.7 ± 0.2 | 29.9 ± 0.7 | 11.9 ± 0.7 | 0.1 ± 0.1 | 0.3 ± 0.0 | 1.5 ± 0.1 | 0 | 0 |
| Example 3, untreated | 61.9 ± 3.2 | 5.8 ± 0.3 | 22.8 ± 2.1 | 8.6 ± 0.9 | 0.2 ± 0.1 | 0.2 ± 0.2 | 0.5 ± 0.3 | 0 | 0 |
| Example 3, treated 3 min. with F-24 | 46.0 ± 2.4 | 4.2 ± 0.3 | 34.6 ± 1.7 | 14.5 ± 0.6 | 0.3 ± 0.2 | 0.1 ± 0.1 | 0.4 ± 0.2 | 0 | 0 |
| Example 3, treated 3 min. with ZSM-5 | 28.9 ± 2.7 | 2.0 ± 0.2 | 47.6 ± 2.3 | 15.4 ± 0.3 | 0.4 ± 0.1 | 0.4 ± 0.1 | 4.6 ± 0.1 | 0.3 ± 0 | 0.4 ± 0 |

The increased oxygen, silicon and aluminum, and the decreased carbon and nitrogen found on the treated lens surfaces as compared to the untreated lens surfaces, are consistent with the results expected if the F-24 or ZSM-5 partially coats the lens surfaces.

Example 18

ACUVUE® OASYS® Brand Contact Lenses with Hydraclear® Plus were removed from their packages and were placed without rinsing into a 1% (wt) dispersion of powdered nanoclay hydrophilic bentonite (Cat. No. 682659 from Aldrich Chemistry) in borate buffered saline solution for 3 minutes at room temperature with stirring. After rinsing in borate buffered saline, the lenses were free of any visible residual surface film, and thus did not require a digital rub. The sessile drop contact angle were determined to be 17.6 ± 2.1°, compared to 50.1 ± 2.3° for an untreated control lens.

Example 19

ACUVUE® OASYS® Brand Contact Lenses with Hydraclear® Plus were removed from their packages and rinsed three times in deionized water. They were not placed into tissue capsules. Lenses were stirred in a 1% (wt) dispersion of powdered nanoclay hydrophilic bentonite (Cat. No. 682659 from Aldrich Chemistry) in deionized water for 3 minutes at room temperature with stirring. After rinsing in borate buffered saline the lenses were clear. Some of these lenses were then placed into a 1% (wt) solution of PVP K-90 in deionized water. The sessile drop contact angles were determined and are shown in Table 10.

Table 10

| Lens | Sessile drop contact angle |
|---|---|
| Untreated control | 50.1 ± 2.3° |
| Example 19 - clay treated | 19.3 ± 2.5° |
| Example 19 - clay and PVP treated | 20.1 ± 2.1° |

Comparative Example

Four contact lenses made from etafilcon A (a polymer that contained no amide or siloxane-containing monomers)

were immersed into a rapidly stirring suspension of 9% (wt) of F-24 in deionized water at 90°C. The lenses were rinsed in borate buffered saline. Their sessile drop contact angle was determined to be 99.8 ± 7.9°, compared to 85.9 ± 7.6° for untreated control etafilcon lenses.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A contact lens comprising at least one clay and at least one silicone component, wherein said at least one clay is applied to the surface of said contact lens and said contact lens does not comprise any diffusible material whose release from said contact lens is inhibited by said at least one clay.

2. A contact lens of claim 1, wherein said contact lens does not comprise any diffusible material.

3. A contact lens of claim 1, wherein said at least one clay comprises aluminum silicate, or wherein said at least one clay is selected from the group consisting of kaolins, smectites, illites, chlorites, sepiolites; zeolites, and attapulgites.

4. A contact lens of claim 1 or 2, wherein said at least one clay is selected from the group consisting of bentonites and zeolites.

5. A contact lens of claim 1, 2, or 4, wherein said at least one silicone component is selected from compounds of Formula I:

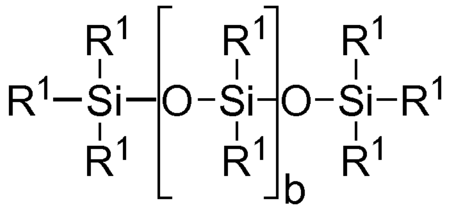

Formula I

wherein:

$R^1$ is independently selected from reactive groups, monovalent alkyl groups, or monovalent aryl groups, any of the foregoing which may further comprise functionality selected from hydroxy, amino, oxa, carboxy, alkyl carboxy, alkoxy, amido, carbamate, carbonate, halogen or combinations thereof; and monovalent siloxane chains comprising 1-100 Si-O repeat units which may further comprise functionality selected from alkyl, hydroxy, amino, oxa, carboxy, alkyl carboxy, alkoxy, amido, carbamate, halogen or combinations thereof;

where b = 0 to 500, where it is understood that when b is other than 0, b is a distribution having a mode equal to a stated value; and

wherein at least one $R^1$ comprises a reactive group.

6. A contact lens of claim 1, wherein said at least one silicone component is selected from the group consisting of monomethacryloxypropyl terminated, mono-n-alkyl terminated polydialkylsiloxane; bis-3-acryloxy-2-hydroxypropyloxypropyl polydialkylsiloxane; methacryloxypropyl-terminated polydialkylsiloxane; mono-(3-methacryloxy-2-hydroxypropyloxy)propyl terminated, mono-alkyl terminated polydialkylsiloxane; and mixtures thereof, or wherein said at least one silicone component is selected from monomethacrylate terminated polydimethylsiloxanes; bis-3-acryloxy-2-hydroxypropyloxypropyl polydialkylsiloxane; mono-(3-methacryloxy-2-hydroxypropyloxy)propyl terminated, mono-butyl terminated polydialkylsiloxane; 2-hydroxy-3-methacryloxypropyloxypropyl-tris(trimethylsiloxy)silane, 3-methacryloxy-2-hydroxypropoxy)propyl-bis(trimethylsiloxy)methylsilane, and 3-methacryloxypropyltris(trimethylsiloxy)silane; and mixtures thereof.

7. A contact lens of claim 1, 2 or 4, wherein said at least one silicone component is selected from mono-(3-methacryloxy-2-hydroxypropyloxy) propyl terminated, mono-butyl terminated polydialkylsiloxane and monomethacryloxypropyl

terminated mono-n-butyl terminated polydimethylsiloxanes, and mixtures thereof.

8. A method of improving the wettability of a contact lens, said method comprising applying, without pretreatment, at least one clay to at least a portion of at least one surface of said contact lens, wherein said contact lens comprises at least one silicone component.

9. A method of comprising applying, without pretreatment, at least one clay to at least a portion of at least one surface of a contact lens comprising at least one silicone component.

10. The method of claim 8 or 9, wherein said contact lens does not comprise any diffusible material.

11. The method of claim 8 or 9 wherein said contact lens is not contacted with a positively charged polyelectrolyte prior to applying said clay.

12. The method of claim 8 or 9 wherein said clay is an aluminum silicate.

13. The method of claim 12 wherein:

    a) said aluminum silicates is selected from the group consisting of kaolins, such as kaolinite, dickite, halloysite, and nacrite; smectites, pyrophyillites, talcs, vermiculites, saucites, nontronites, and saponites; illites; chlorites; sepiolites; zeolites; attapulgites and mixtures thereof,
    b) said clay is a montmorillonite clay,
    c) said clay comprises of particles less than 25 microns in diameter, or
    d) said clay included in a solution in amounts between about 0.001% and about 10 wt% or more specifically wherein said clay is present in said solution in amounts between about 0.01 and about 5 wt%.

14. The method of claim 8 wherein said applying step comprising contacting, for less than one hour, said contact lens with an aqueous dispersion comprising said clay.

15. The method of claim 8 further comprising, after said clay has been applied thereto, packaging said contact lens a buffered aqueous solution in a sealed package, and autoclaving said packaged contact lens.

**Patentansprüche**

1. Kontaktlinse, umfassend mindestens einen Ton und mindestens eine Silikonkomponente, wobei der mindestens eine Ton auf die Oberfläche der Kontaktlinse aufgebracht ist und die Kontaktlinse kein diffusionsfähiges Material umfasst, dessen Freisetzung aus der Kontaktlinse durch den mindestens einen Ton inhibiert wird.

2. Kontaktlinse nach Anspruch 1, wobei die Kontaktlinse kein diffusionsfähiges Material umfasst.

3. Kontaktlinse nach Anspruch 1, wobei der mindestens eine Ton Aluminiumsilikat umfasst oder wobei der mindestens eine Ton aus der Gruppe bestehend aus Kaolinen, Smektiten, Illiten, Chloriten, Sepiolithen, Zeolithen und Attapulgiten ausgewählt ist.

4. Kontaktlinse nach Anspruch 1 oder 2, wobei der mindestens eine Ton aus der Gruppe bestehend aus Bentoniten und Zeolithen ausgewählt ist.

5. Kontaktlinse nach Anspruch 1, 2 oder 4, wobei die mindestens eine Silikonkomponente ausgewählt ist aus Verbindungen der Formel I:

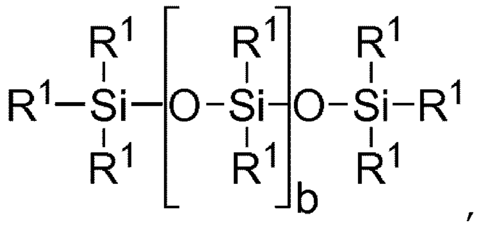

Formel I

wobei:

$R^1$ unabhängig aus reaktiven Gruppen, einwertigen Alkylgruppen oder einwertigen Arylgruppen ausgewählt ist, wobei jede der obigen Gruppen ferner aus Hydroxy, Amino, Oxa, Carboxy, Alkylcarboxy, Alkoxy, Amido, Carbamat, Carbonat, Halogen oder Kombinationen davon ausgewählte Funktionalität umfassen kann; und einwertigen Siloxanketten, die 1-100 Si-O-Wiederholungseinheiten umfassen, die ferner aus Alkyl, Hydroxy, Amino, Oxa, Carboxy, Alkylcarboxy, Alkoxy, Amido, Carbamat, Halogen oder Kombinationen davon ausgewählte Funktionalität umfassen können;
wobei b = 0 bis 500, wobei es sich versteht, dass dann, wenn b von 0 verschieden ist, b eine Verteilung mit einem Modalwert, der einem angegebenen Wert entspricht, ist; und
wobei mindestens ein $R^1$ eine reaktive Gruppe umfasst.

6. Kontaktlinse nach Anspruch 1, wobei die mindestens eine Silikonkomponente aus der Gruppe bestehend aus monomethacryloxypropylterminiertem, mono-n-alkylterminiertem Polydialkylsiloxan; Bis-3-acryloxy-2-hydroxypropyloxypropylpolydialkyl-siloxan; methacryloxypropylterminiertem Polydialkylsiloxan; mono(3-(methacryloxy-2-hydroxy-propyloxy)propylterminiertem, monoalkylterminiertem Polydialkylsiloxan und Mischungen davon ausgewählt ist oder wobei die mindestens eine Silikonkomponente aus monomethacrylatterminierten Polydimethylsiloxanen; Bis-3-acryloxy-2-hydroxypropyloxypropylpolydialkylsiloxan; mono(3-methacryloxy-2-hydroxypropyloxy)propylterminiertem, monobutylterminiertem Polydialkylsiloxan; 2-Hydroxy-3-methacryloxypropyloxypropyltris(trimethylsiloxy)silan, 3-Methacryloxy-2-hydroxypropoxy)propylbis(trimethylsiloxy)methylsilan und 3-Methacryloxypropyl-tris(trimethylsiloxy)silan und Mischungen davon ausgewählt ist.

7. Kontaktlinse nach Anspruch 1, 2 oder 4, wobei die mindestens eine Silikonkomponente aus mono(3-methacryloxy-2-hydroxypropyloxy)propylterminiertem, mono-butylterminiertem Polydialkylsiloxan und monomethacryloxypropylterminierten mono-n-butylterminierten Polydimethylsiloxanen und Mischungen davon ausgewählt ist.

8. Verfahren zur Verbesserung der Benetzbarkeit eine Kontaktlinse, bei dem man ohne Vorbehandlung mindestens einen Ton auf mindestens einen Teil mindestens einer Oberfläche der Kontaktlinse aufbringt, wobei die Kontaktlinse mindestens eine Silikonkomponente umfasst.

9. Verfahren, bei dem man ohne Vorbehandlung mindestens einen Ton auf mindestens einen Teil mindestens einer Oberfläche einer Kontaktlinse, die mindestens eine Silikonkomponente umfasst, aufbringt.

10. Verfahren nach Anspruch 8 oder 9, bei dem die Kontaktlinse kein diffusionsfähiges Material umfasst.

11. Verfahren nach Anspruch 8 oder 9, bei dem die Kontaktlinse nicht vor dem Aufbringen des Tons mit einem positiv geladenen Polyelektrolyt in Berührung gebracht wird.

12. Verfahren nach Anspruch 8 oder 9, bei dem es sich bei dem Ton um ein Aluminiumsilikat handelt.

13. Verfahren nach Anspruch 12, bei dem:

a) das Aluminiumsilikat aus der Gruppe bestehend aus Kaolinen, wie Kaolinit, Dickit, Halloysit und Nacrit; Smektiten, Pyrophylliten, Talken, Vermiculiten, Sauciten, Nontroniten und Saponiten; Illiten; Chloriten; Sepiolithen; Zeolithen; Attapulgiten und Mischungen davon ausgewählt wird,
b) es sich bei dem Ton um einen Montmorillonit-Ton handelt,
c) der Ton Teilchen mit einem Durchmesser von weniger als 25 Mikron umfasst oder
d) der Ton in einer Lösung in Mengen zwischen etwa 0,001 % und etwa 10 Gew. -% enthalten ist oder spezieller

bei dem der Ton in der Lösung in Mengen zwischen etwa 0,01 und etwa 5 Gew. -% vorliegt.

**14.** Verfahren nach Anspruch 8, bei dem der Aufbringungsschritt das Inberührungbringen der Kontaktlinse mit einer wässrigen Lösung, die den Ton umfasst, über einen Zeitraum von weniger als einer Stunde umfasst.

**15.** Verfahren nach Anspruch 8, bei dem man ferner die Kontaktlinse nach dem Aufbringen des Tons darauf in einer gepufferten wässrigen Lösung in einer versiegelten Packung verpackt und die verpackte Kontaktlinse autoklaviert.

**Revendications**

**1.** Lentille de contact comprenant au moins une argile et au moins un composant siliconé, où ladite au moins une argile est appliquée à la surface de ladite lentille de contact et ladite lentille de contact ne comprend aucun matériau diffusable dont la libération depuis ladite lentille de contact est inhibée par ladite au moins une argile.

**2.** Lentille de contact selon la revendication 1, où ladite lentille de contact ne comprend aucun matériau diffusable.

**3.** Lentille de contact selon la revendication 1, où ladite au moins une argile comprend du silicate d'aluminium, ou où ladite au moins une argile est choisie dans le groupe constitué par les suivantes : kaolins, smectites, illites, chlorites, sépiolites, zéolithes et attapulgites.

**4.** Lentille de contact selon la revendication 1 ou 2, où ladite au moins une argile est choisie dans le groupe constitué par les bentonites et les zéolithes.

**5.** Lentille de contact selon la revendication 1, 2 ou 4, où ledit au moins un composant siliconé est choisi parmi les composés de Formule I :

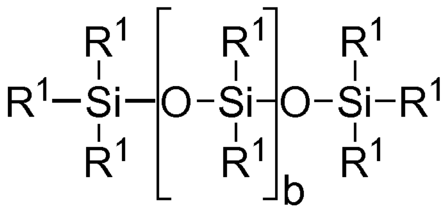

Formule I

où :

$R^1$ est indépendamment choisi parmi les groupements réactifs, les groupements alkyle monovalents ou les groupements aryle monovalents, n'importe lequel des groupements précédents comportant en outre une fonctionnalité choisie parmi hydroxy, amino, oxa, carboxy, alkylcarboxy, alkoxy, amido, carbamate, carbonate, halogène ou des combinaisons de celles-ci ; et des chaînes siloxane monovalentes comprenant 1 à 100 motifs de répétition Si-0 qui peuvent en outre comporter une fonctionnalité choisie parmi alkyle, hydroxy, amino, oxa, carboxy, alkylcarboxy, alkoxy, amido, carbamate, halogène ou des combinaisons de celles-ci ;
où b = 0 à 500, où il est entendu que lorsque b est différent de 0, b est une distribution ayant un mode égal à une valeur donnée ; et
où au moins un radical $R^1$ comprend un groupement réactif.

**6.** Lentille de contact selon la revendication 1, où ledit au moins un composant siliconé est choisi dans le groupe constitué par les suivants : polydialkylsiloxane à terminaison monométhacryloxypropyle, à terminaison mono-n-alkyle ; bis-3-acryloxy-2-hydroxypropyloxypropylpolydialkylsiloxane ; polydialkylsiloxane à terminaison méthacryloxypropyle ; polydialkylsiloxane à terminaison mono-(3-méthacryloxy-2-hydroxypropyloxy)propyle, à terminaison mono-alkyle ; et leurs mélanges, ou où ledit au moins un composant siliconé est choisi parmi les suivante polydiméthylsiloxanes à terminaison monométhacrylate ; bis-3-acryloxy-2-hydroxypropyloxypropylpolydialkylsiloxane ; polydialkylsiloxane à terminaison mono-(3-méthacryloxy-2- hydroxypropyloxy)propyle, à terminaison mono-butyle ; 2-hydroxy-3-méthacryloxypropyloxypropyl-tris(triméthylsiloxy)silane, 3-méthacryloxy-2-hydroxypropoxy)propyl-bis(triméthylsiloxy)méthylsilane et 3-méthacryloxypropyltris(triméthylsiloxy)silane ; et leurs mélanges.

**7.** Lentilles de contact en selon la revendication 1, 2 ou 4, où ledit au moins un composant siliconé est choisi parmi les suivants : polydialkylsiloxane à terminaison mono-(3-méthacryloxy-2-hydroxypropyloxy)propyle, à terminaison mono-butyle et polydiméthylsiloxanes à terminaison monométhacryloxypropyle, à terminaison mono-n-butyle, et leurs mélanges.

**8.** Procédé d'amélioration de la mouillabilité d'une lentille de contact, ledit procédé comprenant l'application, sans prétraitement, d'au moins une argile à au moins une partie d'au moins une surface de ladite lentille de contact, où ladite lentille de contact comprend au moins un composant siliconé.

**9.** Procédé comprenant l'application, sans prétraitement, d'au moins une argile à au moins une partie d'au moins une surface d'une lentille de contact comprenant au moins un composant siliconé.

**10.** Procédé selon la revendication 8 ou 9, où ladite lentille de contact ne comprend aucun matériau diffusable.

**11.** Procédé selon la revendication 8 ou 9, où ladite lentille de contact n'est pas mise en contact avec un polyélectrolyte positivement chargé avant l'application de ladite argile.

**12.** Procédé selon la revendication 8 ou 9, où ladite argile est un silicate d'aluminium.

**13.** Procédé selon la revendication 12, ou

a) lesdits silicates d'aluminium sont choisis dans le groupe constitué par les kaolins, tels que kaolinite, dickite, halloysite, et nacrite ; les smectites, pyrophyillites, talcs, vermiculites, saucites, nontronites et saponites ; les illites ; les chlorites ; les sépiolites ; les zéolithes ; les attapulgites et leurs mélanges,
b) ladite argile est une argile de montmorillonite,
c) ladite argile est constituée de particules de diamètre inférieur à 25 microns, ou
d) ladite argile est incluse dans une solution à des teneurs comprise entre environ 0,001 % et environ 10 % en masse, ou plus spécifiquement, où ladite argile est présente dans ladite solution à des teneurs comprises entre environ 0,01 et environ 5 % en masse.

**14.** Procédé selon la revendication 8, où ladite étape d'application comprend la mise en contact, pendant moins d'une heure, de ladite lentille de contact avec une dispersion aqueuse comprenant ladite argile.

**15.** Procédé selon la revendication 8, comprenant en outre, après l'application de ladite argile, le conditionnement de ladite lentille de contact dans une solution tamponnée aqueuse dans un emballage scellé, et le traitement à l'autoclave de ladite lentille de contact conditionnée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 18757814 A
- US 61786903 A
- US 7052131 B
- WO 02096477 A2
- EP 2253590 A1
- US 7666461 B
- US 3808178 A
- US 4120570 A
- US 4136250 A
- US 4139513 A
- US 4139692 A
- US 4153641 A
- US 4740533 A
- US 5034461 A
- US 5070215 A
- US 5260000 A
- US 5358995 A
- US 5760100 A
- US 5962548 A
- US 5998498 A
- US 6367929 B
- US 6849671 B
- US 6943203 B
- US 7521488 B
- US 7825170 B
- US 7939579 B
- EP 080539 A
- WO 9631792 A
- US 5314960 A
- US 5331067 A
- US 5244981 A
- US 5371147 A
- US 5321108 A
- US 5387662 A
- US 5539016 A
- US 20020016383 A
- US 3408429 A
- US 3660545 A
- US 4113224 A
- US 4197266 A
- US 20060229423 A
- US 7691916 B